Europäisches Patentamt

European Patent Office

Office européen des brevets

(11) Publication number: **0 455 093 A2**

# EUROPEAN PATENT APPLICATION

(21) Application number: **91106466.5**

(22) Date of filing: **22.04.91**

(51) Int. Cl.⁵: **A61K 37/02**, //(A61K37/02, A61N5:00)

(30) Priority: **23.04.90 US 511281**

(43) Date of publication of application:
**06.11.91 Bulletin 91/45**

(84) Designated Contracting States:
**AT BE CH DE DK ES FR GB GR IT LI LU NL SE**

(71) Applicant: **YEDA RESEARCH AND DEVELOPMENT COMPANY LIMITED**
**P.O. Box 95**
**Rehovot 76100(IL)**

(72) Inventor: **Schwartz, Michal, Weizmann Institute of Science**
**Neve Metz 10**
**Rehovot(IL)**

(74) Representative: **Vossius & Partner**
**Siebertstrasse 4 P.O. Box 86 07 67**
**W-8000 München 86(DE)**

(54) **Use of a tumor necrosis factor for facilitating nerve regeneration.**

(57) Administration of a Tumor Necrosis Factor to the site of injury of central nervous system nerve axons in mammals will result in induction and facilitation of the regeneration of axons across the site of the injury. Recombinant human tumor necrosis factor alpha is the preferred substance for such use.

EP 0 455 093 A2

The present invention relates to the use of a Tumor Necrosis Factor (TNF) for facilitating and enhancing regeneration of axons of injured nerves of the central nervous system (CNS) in mammals.

Mammalian CNS neurons have a negligible capacity to regenerate after lesions. In contrast, neurons in the CNS of lower vertebrates and in the peripheral nervous system of mammals are endowed with a high post-traumatic capacity to regenerate. In the goldfish visual system, the retinal ganglion cells regenerate several axons and make functional connections with their appropriate targets. The regenerated axons become myelinated and form their normal pattern of synaptic contacts with their targets. In mammals, however, optic nerve injury leads to the death of most of the axotomized neurons and the failure of the surviving cells to regrow their axons.

The differences in regenerative capacity have been attributed to the different compositions of the respective cellular environments and to different responses to injury displayed by the non-neuronal cells, ranging from supportive and permissive to nonsupportive and hostile for regeneration. The same cell type may support or inhibit regeneration, depending on its state of maturity or differentiation. Astrocytes and oligodendrocytes are examples of cells in which such a dichotomy is manifested. In developing and in spontaneously regenerating nerves, these cells support (astrocytes) and permit (oligodendrocytes) growth. However, in nonregenerating adult mammalian nerves, astrocytes form the nonsupportive scar tissue and the mature oligodendrocytes inhibit axonal growth. Maturation of these cells may be regulated differently during development than after injury.

Schnell, L. and Schwab, M.E., Nature, **343:** 269-272 (1990), report that CNS white matter, cultured oligodendrocytes (the myelin producing cells of the CNS), and CNS myelin itself are strong inhibitors of neuron growth in culture, a property associated with defined membrane-bound surface proteins on myelin and oligodendrocytes. Monoclonal antibodies which neutralize the inhibitory effect of these proteins were implanted intracerebrally into rats. After transection of the cortico-spinal tract, massive sprouting occurred at the lesion site and fine axons and fascicles were observed up to 7-11 mm from the lesion.

Axotomized mammalian central neurons have been shown to regenerate their axons over long distances, if special conditions are provided by replacement of the optic nerve by a segment of a peripheral autologous nerve.

Prior work of the present inventor and others has shown that the CNS of lower vertebrates, specifically regenerating fish optic nerve, is a source of factors which, when applied at the appropriate time and in appropriate amounts to injured mammalian adult optic nerves, can support regenerative axonal growth. See Schwartz et al, Science **228:** 600-603 (1985); Hadani et al, Proc. Natl. Acad. Sci. USA, **81:** 7965-69 (1984); Lavie et al, Brain Res., **419:** 166-173 (1987); Solomon et al, Metab. Pediatr. Syst. Ophthalmol., **11:** 1-2, 31-2 (1988); and Cohen et al, Neurosci. Res., **22:** 269-273 (1989).

TNF, a factor produced mainly by activated macrophages, and originally characterized as having anti-tumor activity has been found to be a multipotent cytokine . It has a pleiotropic activity both in vitro and in vivo (for reviews see Beutler, B. and Cerami, A., New Engl. J. Med. **316:** 379-385 (1987); Old, L.J., Sci. Am. **258:** 59-75 (1988)) including cytotoxic effects against chemically transformed tumor cells (Carswell, E.A. et al., Proc. Natl. Acad. Sci. USA, **72:** 3666-3670 (1975); Sugarman, B.J. et al., Science, **230:** 943-945 (1985)), stimulation of IL-1 secretion (Bachwich, P.R. et al., Biochem. Biophys. Res. Commun. **136:** 94-101 (1986)), stimulation of prostaglandin E and collagenase production (Dayer, J.M. et al., J. Exp. Med. **162:** 2163-2168 (1985)), inhibition of lipogenic gene expression in adipocytes (Torti, F.M. et al., Science, **229:** 867-869 (1985)) and stimulation of various immune effector cells (Shalaby, M.R. et al., J.Immunol. **135:** 2069-2073 (1985)). In spite of extensive studies, little is known about the physiological role of TNF in general, and in nonmalignant situations in particular.

In contrast to the broad spectrum of activities of TNF in the immune system, little is known about the role of TNF in the brain. While in the immune system, TNF-α is a product of macrophages, indication has been provided that in the brain TNF might be produced by microglia (Sawada, M. et al., Brain Res. **491:** 394-397 (1989)), and also by astrocytes (Robbins, D.S. et al.,J. Immunol., **139:** 2593-2597 (1987)). A role for TNF-α in immune-mediated demyelination in multiple sclerosis (MS) has previously been hypothesized on the basis of primarily experimental evidence (Brosnan, C.F. et al., J. Neuroimmunol., **18:** 87-94 (1988)). Robbins, D.S. et al, see above, have reported that stimulation of rat astrocytes in vitro resulted in the generation of a cytotoxic factor that is functionally similar to TNF, and that recombinant human tumor necrosis factor (rhTNF) has cytotoxic activity directed against rat oligodendrocytes. Selmaj, K.W. et al., Ann.Neurol., **23:** 339-346 (1988) reported on the testing of rhTNF for its effect on myelinated cultures of mouse spinal cord tissue, finding that it induced delayed-onset oligodendrocyte necrosis and a type of myelin dilatation. None of these references appear to describe specific activity of TNF in nerve tissues in vivo.

Despite substantial research efforts worldwide, no safe and effective means for causing CNS regeneration in mammals, and particularly in humans, has yet been developed. Such a means, and particularly a pharmaceutical which can be injected at the site of desired regeneration would be greatly desirable in order to alleviate post-traumatic paraplegia or quadraplegia, blindness, deafness, surgically associated axotomy, etc. Accordingly, the technical problem underlying the present invention was to provide compounds for use in the preparation of pharmaceutical compositions for causing CNS regeneration in mammals and particularly in humans.

It has now been found according to the present invention that TNF will facilitate axonal regeneration. Accordingly, the present invention provides use of a Tumor Necrosis Factor in the preparation of a pharmaceutical composition for induction and facilitation of the regeneration of axons of injured nerves of the CNS in mammals, particularly in humans.

It is also an object of the present invention to provide a pharmaceutical composition comprising a unit dose of a TNF and a pharmaceutically acceptable carrier or excipient in a quantity effective for induction and facilitation of the regeneration of injured axons of the CNS in mammals when administered to the site of the injury.

It is a further object of the present invention to provide a method for inducing and facilitating the regeneration of axons of injured nerves of the central nervous system in a mammal comprising administering to the site of the injury an effective quantity of a Tumor Necrosis Factor.

The figures show

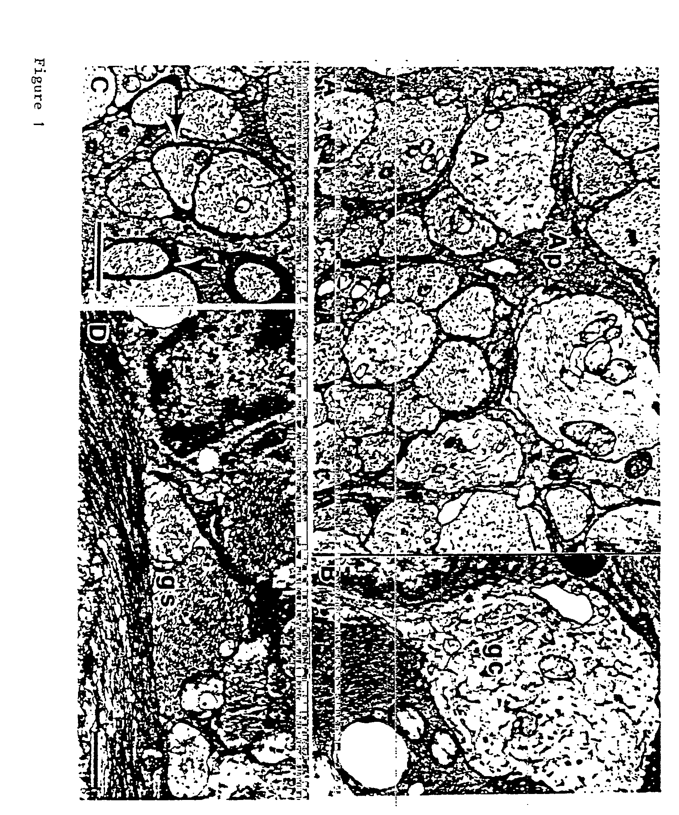

Figure 1A - 1D: electron micrographs from a cross-section of experimental TNF-treated nerves, from an area within the second millimeter distal to the site of injury, showing the characteristics of newly growing axons. Fig. 1A shows abundant unmyelinated axons embedded in a typical astrocytic environment; Fig. 1B shows structures resembling growth cones embedded in astrocytic processes; Fig. 1C shows unmyelinated axons in an early process of remyelination by dark cytoplasm of oligodendrocytes; and Fig. 1D shows control injured but not treated nerves, the area being completely degenerative, containing astrocytic processes and degenerating axons. The identified portions are axons (Ax), astrocytic process (Ap), growth cone (gc) and glial scar (gs).

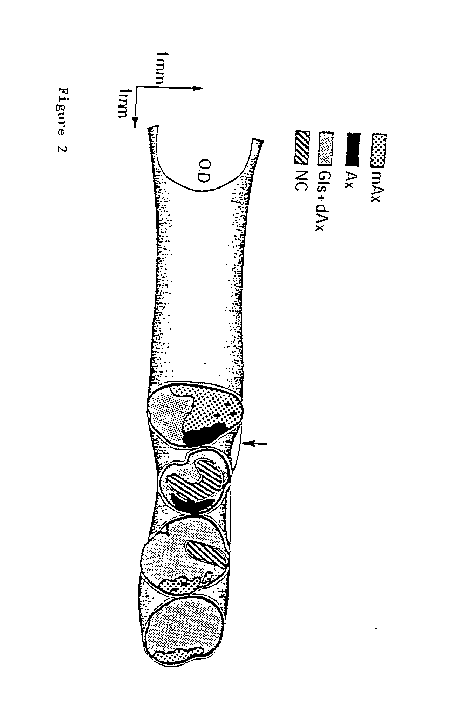

Figure 2 : three-dimensional reconstruction of TNF-treated nerves, 6 weeks postoperatively. Thin sections of adult rabbit optic nerve were cut, mounted on grids, photographed at x 140 and montages were constructed at each level. The compartments that could be identified in each cross-section contained newly growing axons (Ax) and myelinated axons (mAx); nitrocellulose (NC); and glial scar and degenerating axons (GLS + dAx).

Regeneration of axons at the site of a CNS injury is induced and facilitated by the administration to the area of the injury of a Tumor Necrosis Factor (TNF). The TNF seems not to affect directly the growth of the injured axons, but rather to affect the cellular environment so as to make it permissive to axon growth leading to nerve regeneration.

While it is preferred that the TNF be of the same species as the mammal being treated, this is not critical. Muteins or modified TNF molecules having amino acid residues added, deleted or substituted as compared to native human TNF, as well as TNF derivatives which have added moieties or other peptide sequences to improve their physical properties for use in a pharmaceutical composition, are encompassed for use in the present invention as long as such modified proteins maintain the function of TNF which is used in accordance with the present invention to facilitate regeneration of CNS axons. The applicability of any such TNF-variant may be readily tested, without undue experimentation, by means of an in vitro test for selective oligodendrocyte cytotoxicity, as described in Sivron, T. et al., Glia **3**: 267-276 (1990).

Thus, when the term "a Tumor Necrosis Factor" is used throughout the present specification and claims, it is understood to include native TNF of any species, recombinant TNF of any species and modified TNF, which have the property of inducing and facilitating the regeneration of injured mammalian axons in vivo, which function may be predicted by means of the in vitro test for selective cytotoxicity to oligodendrocytes. In a preferred embodiment, human recombinant TNF-$\alpha$ is used according to the invention.

The TNF may be obtained by any convenient technique. For example, it may be obtained by the process of Mathews et al, Br. J. Cancer, **42**: 416-422 (1980), or of Green et al, J. Natl. Cancer Inst., **59**: 5, 1519-1522 (1977). The TNF is preferably obtained by recombinant DNA technologies. (Pennica et al., Nature **312**: 724-729 (1984); Shirai et al., Nature **313**: 803-806 (1985); European Patent Applications EP 155,549, EP 158,286, EP 168,214 and others). Recombinant human tumor necrosis factor (rhTNF) is presently commercially available.

The TNF is used in a quantity and purity sufficient to facilitate regeneration of axons of injured nerves in mammals, particularly humans. The TNF is administered in any manner which is suitable to bring it to the vicinity of the injured axons to be regenerated. Preferably, the TNF is injected in a pharmaceutically acceptable liquid carrier directly to the site. Alternatively, an implant bearing the TNF may be surgically inserted. Such an implant may consist of any material, such as nitrocellulose, which will absorb TNF like a sponge and slowly release it at site of implantation. Other means of delivery will be apparent to those skilled in this art and are intended to be comprehended within the scope of the present invention.

The amount of the TNF to be administered to any given patient depends on a variety of factors, such as the injury being treated, the site of injured axons it is wished to regenerate and the condition of the patient. Typically, however, the TNF is administered as a single injection or soaked onto nitrocellulose or any other adsorbable carrier. Precise dosages will be empirically determined.

The TNF is preferably administered as soon as possible after the injury being treated occurs. Thus, it is preferably used for acute injury rather than chronic injury. It is presently believed that it might be more difficult to facilitate regeneration in accordance with the present invention the longer a period of degeneration has existed, but it is not ruled out that chronic situations may also be treated according to the invention.

While the administration of TNF alone shows good results, such treatment may be combined with any type of concomitant therapy which may tend to augment its effects. For example, combination with other factors inducing and facilitating regeneration of injured axons, such as Interleukin-2, or irradiation of the injury site with low energy laser, preferably He-Ne laser irradiation (632.8 nm 35 mW,), that delays or prevents the post-traumatic processes of degeneration of the nerves in mammals (Assia, E. et al, Brain Res., **476**: 205-212 (1989)), thus facilitating the TNF effect in promoting axonal growth and regeneration.

The various injuries which can be treated in accordance with the present invention are myriad and will be readily apparent to those of ordinary skill in the art. Without limitation, there may be mentioned neural trauma, diseases involving acute or subacute damage to CNS axon caused by pressure or ischemia, e.g. glaucoma, anterior ischemic optic neuropathy, spinal cord injuries, injuries to the optic nerve or to the aural nerves, etc. Injury to CNS neurons during neurosurgery or caused by tumors may also be treated by means of the present invention.

For the purpose of the present invention, the TNF may be formulated with any pharmaceutically acceptable carrier or diluent. The TNF may be presented as an aqueous solution, for example as a sterile aqueous solution. A solution or powder containing TNF may be stabilized by means of a stabilizing agent. It may be formulated in a unit dosage injectable form (solution, suspension, emulsion) preferably in a pharmaceutically acceptable carrier medium that is inherently non-toxic and non-therapeutic. Examples of such vehicles include saline, Ringer's solution, dextrose solution, mannitol and normal serum albumin. Non-aqueous vehicles such as fixed oils and ethyl oleate may also be used. The carrier medium may contain minor amounts of additives such as substances that enhance isotonicity, solubility and/or chemical stability, e.g., buffers, detergents and preservatives. Various formulations for TNF are already known for other indications. Such formulations may also be used for the purpose of the present invention as long as the desired function of the TNF is not affected thereby.

The invention will now be illustrated by the following non-limitative examples:

## Example 1

Adult rabbits (albino, Weizmann Institute Animal House) were deeply anesthetized with xylazine (5 mg/kg) and ketamine (35 mg/kg). The left optic nerves were exposed as previously described (Solomon et al, J. Neurosci. Meth., **12**: 259-262 (1985)) and transected except for the meningeal membrane at a distance of 5-6 mm from the eyeball using a sharpened needle. In all operated nerves, a piece of nitrocellulose, 3 mm long and 1 mm wide, was inserted at the site of the injury. For the experimental group of rabbits, the nitrocellulose was soaked with rhTNF (100 U/nerve; $6 \times 10^7$ U/mg) for 1 hour prior to the insertion. For the control group, the nitrocellulose was soaked in the medium used to dilute the TNF (DMEM), free of any active substances. Beginning within 30 min of surgery, the experimental animals were also daily treated for ten days with low energy He-Ne laser irradiation (632.8 nm, 35 mW) for 5 min per day.

The experimental and control injured nerves were examined qualitatively and quantitatively at 6 weeks postoperatively by transmission electron microscopy. Such an analysis allows distinction between newly growing axons and spared axons. Quantitative analyses were carried out as follows: systematically selected thin sections were photographed by the electron microscope at low magnification (x 140), which permitted identification of the newly growing axons (including unmyelinated and thinly myelinated axons).

Figure 1 includes photographs taken from a cross-section of experimental TNF-treated nerves, from an area within the second millimeter from the site of injury. These pictures depict characteristics of newly growing axons, including abundant unmyelinated axons embedded in a typical astrocytic environment (Fig. 1A), structures resembling growth cones embedded in astrocytic processes (Fig. 1B), and axons in early stages of myelination by oligodendrocyte dark cytoplasm (Fig. 1C). The unmyelinated axons were counted in four regions along the nerve: 1 mm before the site of injury, at the end of the nitrocellulose, 2 mm distally to it, and at the area deep into the distal stump. Table 1 shows the obtained results.

## Table I

## Number of unmyelinated and myelinated axons in a

## TNF-treated nerve

Distance from the globe          Number of counted axons***

| (mm) | Unmyelinated | Myelinated | Total |
|------|--------------|------------|-------|
| 6    | 7530         | 31357      | 38887 |
| 8    | 8466         | 591        | 9057  |
| 10*  | 200          | 2272       | 2472  |
| 12   | -            | 2732       | 2732  |

The results represent the analysis of one nerve by the sampling method. These results were qualitatively reproducible in 3 additional nerves.

*    The last region in which nitrocellulose could be observed.

**   The site of injury located between the 6 and 7 mm distally to the globe.

***  In control animals, no unmyelinated axons could be observed in the section taken at the same distance as in the experiment.

The maximal number of unmyelinated axons is seen within the second millimeter distal to the site of injury (8466). In control untreated animals, at the same distance from the lesion, no unmyelinated or myelinated axons could be observed. The number of unmyelinated axons decreases with the increase in the distance from the site of the injury. The apparent increase in number of myelinated axons in the area 4 mm distally to the lesion relative to the 2 mm distance, might be due to myelination of part of the growing axons, or axons which were injured but their degeneration had not reached the examined area (4 mm on from the injury). Three-dimensional reconstruction of the nerve (Fig. 2) showed that the area occupied by the viable unmyelinated and myelinated axons, as well as their total number, decreases upon increasing distance from the site of injury, thus emphasizing that the observed unmyelinated axons are, indeed, newly growing axons, rather than spared axons that have lost their myelin sheaths.

**Example 2**

S.P.D. rats, 3 months old weighing 300 g were anesthetized with a loading dose of 15 mg ketamine and 4 mg xylazine 2%. After performing a laminectomy at $T_{12}$ level, the left hemicord was crushed with Weck MICRO-CLIPTM m x 1 mm (catalog No. 065145) for 30 seconds. No gross anatomical disruption was noticed after the crush.

Following the crush, $10\mu l$ (100 U) of TNF were injected at the site of injury, into the cord, after which the wound was clcsed.

Eight weeks later the rats were anesthetized again and after a partial laminectomy at $T_6$ level, $10\mu l$ Horseradish Peroxidase (HRP) 30% were injected into the cord, and the site of injection was sealed by silicone cream. Twenty four hours after the HRP injection, the rats were sacrificed and perfused with paraformaldehyde. The cord was removed and longitudinal sections, 75 microns, were cut. The sections were developed by cobalt intensification as described by M.M. Mesulam, in "Tracing Neural Connections with Horseradish Peroxidase", IBRO Handbook Series, Methods in Neuroscience, John Wiley and Sons, 1982. In the control injured but not TNF-treated nerves, a complete degeneration was observed. In the TNF-treated spinal cord, HRP-stained nerve fibers traversing the site of injury were observed.

The foregoing description of the specific embodiments will so fully reveal the general nature of the invention that others can, by applying current knowledge, readily modify and/or adapt for various applications such specific embodiments without departing from the generic concept, and therefore such adaptations and modifications are intended to be comprehended within the meaning and range of equivalents of the disclosed embodiments. It is to be understood that the phraseology or terminology herein is for the purpose of description and not of limitation.

**Claims**

1. Use of a Tumor Necrosis Factor for the preparation of a pharmaceutical composition for inducing and facilitating the regeneration of injured axons of the central nervous system in mammals when administered to the site of the injury.

2. The use of a Tumor Necrosis Factor according to claim 1 wherein the mammal is a human.

3. The use of a Tumor Necrosis Factor according to claim 1 or 2 wherein the Tumor Necrosis Factor is recombinant human Tumor Necrosis Factor.

4. The use of a Tumor Necrosis Factor according to claim 3 wherein the Tumor Necrosis Factor is recombinant human Tumor Necrosis Factor alpha.

5. The use of a Tumor Necrosis Factor according to any one of claims 1 to 4, wherein the Tumor Necrosis Factor is a modified human Tumor Necrosis Factor.

6. The use of a Tumor Necrosis Factor according to any of claims 1 to 5 wherein the pharmaceutical composition is to be administered by direct injection to the site of the injury.

7. The use of a Tumor Necrosis Factor according to any of claims 1 to 6 for the preparation of a pharmaceutical composition for inducing and facilitating the regeneration of injured spinal cord axons.

8. The use of a Tumor Necrosis Factor according to any of claims 1 to 6 for the preparation of a pharmaceutical composition for inducing and facilitating the regeneration of injured optic nerve axons.

9. The use of a Tumor Necrosis Factor according to any one of claims 1 to 8, wherein the pharmaceutical composition is to be administered together with low energy laser irradiation of the injury site.

10. The use of a Tumor Necrosis Factor according to any of claims 1 to 8 wherein the pharmaceutical composition is to be administered together with Interleukin-2.

11. The use of a Tumor Necrosis Factor for inducing and facilitating the regeneration of injured axons of the central nervous system in mammals when administered to the site of the injury.

12. The use of a Tumor Necrosis Factor according to claim 11 wherein the Tumor Necrosis Factor is recombinant human Tumor Necrosis Factor alpha.

**Claims for the following Contracting State: ES**

1. Method for the preparation of a pharmaceutical composition for inducing and facilitating the regeneration of injured axons of the central nervous system in mammals when administered to the site of the injury which comprises combining a Tumor Necrosis Factor and a pharmaceutically acceptable carrier and/or diluent.

2. The method according to claim 1 wherein the mammal is a human.

3. The method according to claim 1 or 2 wherein the Tumor Necrosis Factor is recombinant human Tumor Necrosis Factor.

4. The method according to claim 3 wherein the Tumor Necrosis Factor is recombinant human Tumor Necrosis Factor alpha.

5. The method according to any one of claims 1 to 4, wherein the Tumor Necrosis Factor is a modified human Tumor Necrosis Factor.

6. The method according to any one of claims 1 to 5, wherein the pharmaceutical composition is to be administered by direct injection to the site of the injury.

7. The method according to any one of claims 1 to 6, wherein the injured axons are injured spinal cord axons.

8. The method according to any one of claims 1 to 6, wherein the injured axons are injured optic nerve axons.

9. The method according to any one of claims 1 to 8, wherein the pharmaceutical composition is to be administered together with low energy laser irradiation of the injury site.

10. The method according to any one of claims 1 to 8, wherein the pharmaceutical composition is to be administered together with Interleukin-2.

Figure 1

Figure 2